# EUROPEAN PATENT APPLICATION

(11) **EP 4 209 273 A1**
(43) Date of publication of application: **12.07.2023**
(21) Application number: 22305016.2
(22) Date of filing: 10.01.2022
(51) Int. Cl.: B01L 3/00, G01N 1/28, C12M 1/00, C12N 1/06, B02C 7/00

(54) **CHIP FOR MICRO-ORGANISM LYSIS, GRINDER AND USE THEREOF**

(71) Applicant: Direct Analysis, 38000 Grenoble (FR)
(72) Inventor: TOUTAIN, Rémi, 38000 Grenoble (FR); KAPPLER, Chloé, 38000 Grenoble (FR)
(74) Representative: Icosa

(57) **Abstract**

The present invention relates to a chip for micro-organism lysis comprising a lysing chamber, a mechanical system operating with such chip and methods of micro-organism lysing.

## Description

### FIELD OF INVENTION

The present invention relates to a chip for micro-organism lysis, a mechanical system operating with such chip and methods of micro-organism lysing.

### BACKGROUND OF INVENTION

Micro-organism lysis is the phenomenon of destruction - partially or totally - of the external protection of the micro-organism. In particular, cellular lysis is the destruction of the cellular membrane. After lysis, the internal content of the micro-organism is released and can be extracted or identified by various methods. Especially, nucleic acid materiel - DNA or RNA - can be released and used as starting material for Polymerase Chain Reaction, leading to identification of the type of micro-organism. Protein material may be also retrieved and used for micro-organism identification. Such methods are now very popular in numerous domains, including diagnostic and quality control in food industry, hygiene or cosmetics.

Various methods for micro-organism lysis are known, and may be classified under three categories. Chemical lysis uses chemicals to degrade the external protection of micro-organism. However, these chemicals may also degrade the valuable internal content of micro-organism. Thermal lysis uses heat to degrade the external protection of micro-organism. But heating is usually leading to degradation of nucleic acid material and proteins. Finally, mechanical lysis aims at disrupting the external protection of micro-organisms by a mechanical stress.

In addition, it is desirable for a device for lysis of micro-organism to be easily transportable, efficient and highly reliable with low volume of sample, and allowing for separation of internal material of micro-organism from external protection.

International patent application WO 2015181743 describes in particular a device for mechanical lysis. In said device, the mechanical lysis is carried out by shearing a suspension of micro-organisms between two walls, one of the two walls having a rough glass bearing surface. However, results obtained, especially on Gram-positive bacteria, are poor and use of glass is not allowed in food and health domains.

European patent application EP 3222989 discloses a mechanical lysis device in which a suspension of micro-organism is grinded on an abrasive surface. However, use of a grinding surface leads to complex procedures and sensitivity of identification is not sufficient.

The subject of this disclosure is a specific chip structure in which various layers are used to define a chamber where mechanical lysis is achieved, and simultaneously allowing for separation of internal material of microorganism. This chip overcomes some or all of the drawback mentioned hereabove.

### SUMMARY

This invention relates to a chip for micro-organism lysing comprising
a) a multilayer stack comprising
   i. a first double-sided adhesive layer comprising a chamber hole;
   ii. a flexible filter layer in direct contact with the first double-sided adhesive layer and covering the chamber hole;
   iii. a second double-sided adhesive layer in direct contact with the flexible filter layer, said second double-sided adhesive layer comprising a chamber hole; and
   iv. a flexible membrane on the first double-sided adhesive layer, opposite the flexible filter;
   wherein all chamber holes are superimposed to form a lysing chamber; and
b) a closing surface on the second double-sided adhesive layer of the multilayer stack, opposite the flexible membrane;
c) an inlet port in fluidic communication with the lysing chamber.

Indeed, this chip allows mechanical lysis of micro-organisms avoiding degradation of its internal content, nucleic acid material and proteins.

Moreover, using a filter allows the lysis of micro-organisms for analysis in food and health domains.

Finally, the chip of the present invention allows a simple mechanical lysis procedure and an increase of the sensitivity of identification.

According to other advantageous aspects of the invention, the chip for micro-organism lysing includes one or more of the following features, taken alone or in any technically possible combination.

The closing surface is a second flexible membrane.

Indeed, the use of a second flexible membrane allows to increase the efficiency of the lysis and to decrease the disparity in reproducibility of the efficiency of the lysis.

The chip for micro-organism lysing further comprises
a) a back cover on the membrane side of the multilayer stack, comprising a pestle hole aligned with the lysing chamber;
b) a rigid cover on the second flexible membrane, opposite the back cover; and
c) fastening means to maintain rigid cover and back cover in contact with the closed multilayer stack;
wherein the inlet port is on the rigid cover; the fluidic communication being arranged by superimposed inlet holes in the layers of the multilayer stack and in the second flexible membrane forming an inlet chamber and an inlet channel between the chamber hole and the inlet hole of one of the double-sided adhesive layers.

Moreover, the back and rigid covers provide with mechanical stability of the chip, allowing easier handling.

Furthermore, arranging the inlet port on the rigid cover allows the connection of cones or syringes for injecting liquid elements.

Moreover, the pestle hole allows to increase the efficiency of the mechanical lysis of the micro-organisms by defining a zone for applying the mechanical action which precisely corresponds to the size of the lysing chamber.

Finally, the use of a rigid cover and fastening means allows to increase the rigidity of the multilayer stack and membranes all over the chip except on the lysing chamber allowing the applied mechanical action to be focused on the lysing chamber.

The chip for micro-organism lysing further comprises an outlet port in fluidic communication with the lysing chamber, the outlet port being on the rigid cover; and
i. the fluidic communication, noted A1, being arranged by superimposed outlet holes in the layers of the multilayer stack and in the second flexible membrane forming an outlet chamber; and an outlet channel between the chamber hole and the outlet hole of the double-sided adhesive devoid of an inlet channel; or
ii. the fluidic communication, noted B 1, being arranged by a communication hole in the second flexible membrane, said communication hole being superimposed on the periphery of the lysing chamber; and an outlet channel between the communication hole and the outlet port in the rigid cover.

Indeed, the inlet and outlet ports being on the same side of the same cover allows to facilitate the manipulations performed on the micro-organism since the simultaneous use of tools in the inlet and outlet ports is performed on the same side of the chip.

Moreover, arranging the outlet port on the rigid cover allows the connection of cones or syringes for collecting liquid elements.

The fastening means comprise a third double-sided adhesive layer in direct contact between the back cover and the multilayer stack, said third double-sided adhesive layer comprising a pestle hole superimposed with the lysing chamber.

The fastening means comprise a third double-sided adhesive layer in direct contact between the back cover and the multilayer stack and comprising a pestle hole superimposed with the lysing chamber; and a fourth double-sided adhesive layer in direct contact between the rigid cover and the second flexible membrane and comprising an inlet hole superimposed with the inlet chamber and
i. an outlet hole superimposed with the other outlet holes for fluidic communication A1; or
ii. a chamber hole superimposed with the lysing chamber for fluidic communication B 1.

The closing surface is a rigid cover.

Indeed, the use of a rigid cover allows to increase the rigidity of the multilayer stack and membranes all over the chip except in the lysis chamber allowing the applied mechanical action to be gathered on the lysis chamber.

Moreover, it allows, when applying a pressure on the flexible membrane, to crush the micro-organisms against a rigid cover increasing the efficiency of the cell lysis.

The chip for micro-organism lysing further comprises
a) a back cover on side of the multilayer stack opposite the rigid cover, comprising a pestle hole aligned with the lysing chamber;
b) fastening means to maintain back cover in contact with the multilayer stack;
c) an outlet port in fluidic communication with the lysing chamber, the outlet port being on the rigid cover; and
   i. the fluidic communication, noted A2, being arranged by superimposed outlet holes in the layers of the multilayer stack forming an outlet chamber; and a outlet channel between the chamber hole and the outlet hole of one of the double-sided adhesive layers; or
   ii. the fluidic communication, noted B2, being arranged by an outlet channel between the chamber hole and the outlet port in the rigid cover;
and wherein the inlet port is on the rigid cover; the fluidic communication being arranged by superimposed inlet holes in the layers of the multilayer stack forming an inlet chamber and an inlet channel between the chamber hole and the inlet hole of a double-sided adhesive layer devoid of an outlet channel.

The at least one flexible membrane is polyethylene terephthalate membrane, preferably at least one flexible membrane has a thickness ranging from 15 µm to 150 µm. More preferably, all flexible membranes are polyethylene terephthalate membrane having a thickness ranging from 15 µm to 150 µm.

Indeed, using polyethylene terephthalate allows to obtain membranes which are biologically compatible.

Moreover, the thickness range is optimized: below 15 µm the membranes are subject to ripping while above 150 µm there is a loss of efficiency.

The flexible filter layer is a polycarbonate layer, preferably flexible filter layer has a porosity ranging from 0,2 µm to 1 µm and / or a thickness ranging from 5 µm to 50 µm.

The invention also relates to a system for micro-organism lysing comprising
a. a chip for micro-organism lysing according to one aspect of the invention,
b. a grinder comprising
   i. a platform with a recess configured to receive the chip for micro-organism lysing;
   ii. a pestle configured to be aligned with the lysing chamber;
   iii. a motor to rotate said pestle; and
   iv. pressuring means to press said pestle against the lysing chamber.

Indeed, this system is compact and the compatibility of the elements is optimized to increase the efficiency of the mechanical lysis.

Moreover, the application of a pressure and a rotation allows to increase the efficiency of the cell lysis by efficiently deforming the cell membrane.

According to other advantageous aspects of the invention, the system for micro-organism lysing includes the following feature: the pestle grinding part comprises a step.

Indeed, this allows to create pinching zones in the lysing chamber allowing the rupture of the cells.

The invention also relates to a method for micro-organism lysing comprising
a. Introducing a sample comprising micro-organisms via the inlet import in a chip for micro-organism lysing according to one aspect of the invention;
b. Pressing the lysing chamber with a rotating pestle; and
c. Recovering the content of cells.

Indeed, the simultaneous application of a pressure and a rotation allows to increase the efficiency of the cell lysis by efficiently deforming the cell membrane.

According to other advantageous aspects of the invention, the method for micro-organism lysing includes one or more of the following features, taken alone or in any technically possible combination.

The chip for micro-organism lysing is placed in the recess of the platform of a grinder, said grinder comprising
i. a pestle configured to be aligned with the lysing chamber of the chip for micro-organism lysing;
ii. a motor to rotate said pestle; and
iii. pressuring means to press said pestle against the lysing chamber.

Indeed, the alignment of the pestle with the lysing chamber allows to efficiently distribute the pressure over the lysing chamber.

The force of pestle is ranging from 1 N to 24 N and/or the rotational speed of pestle is ranging from 30 rpm to 150 rpm.

This force and rotational speed range allows the increase of the efficiency of the cells lysis without damaging the cell content.

### DEFINITIONS

In the present invention, the following terms have the following meanings:

**"Direct contact"** between two layers means that both layers are not separated by another layer.

**"Flexible"** refers to elements of the chip, in particular filter and membranes, that are able to deform under pressure in the direction normal to their surface (bending), and are able to deform within their surface and form pleats (stretching and folding) when sheared.

**"Micro-organism"** refers to biological entities such as viruses, archaea, bacteria, fungi and cells.

**"On"** in relationship with two layers, means that one layer is laid on the other layer, either in **direct contact** or separated by one or several intermediate layers.

### DETAILED DESCRIPTION

This disclosure relates to a chip 100 for micro-organism lysing. In a first basic configuration, the chip 100 comprises a multilayer stack 110 comprising a first double-sided adhesive layer 111 comprising a chamber hole. A flexible filter layer 112 is in direct contact with the first double-sided adhesive layer 111. The flexible filter layer 112 covers the chamber hole. A second double-sided adhesive layer 111 is in direct contact with the flexible filter layer 112. The second double-sided adhesive layer 111 comprises a chamber hole. Last, a flexible membrane 113 is on the first double-sided adhesive layer 111, opposite the flexible filter layer 112. In addition, all chamber holes are superimposed to form a lysing chamber 120.

The chip 100 further comprises a closing surface on the second double-sided adhesive layer 111 of the multilayer stack 110, opposite the flexible membrane 113. And an inlet port 130 is in fluidic communication with the lysing chamber 120.

In this first basic configuration, the chip 100 comprises two outer surfaces - the flexible membrane 113 on one side and the closing surface on the opposite side - inside which two adhesive layers 111 sandwich the flexible filter layer 112. The thickness of the double-sided adhesive layers 111 and the chamber holes define a hollow space in which the sample can be introduced through the inlet port 130. The combination of the multilayer stack 110 with the closing surface is thereafter a closed multilayer stack 110.

In this disclosure, filter layer 112 and membrane 113 are flexible. By flexible, it is meant that these flat elements are able to deform under pressure in the direction normal to their surface (bending), and are able to deform within their surface and form pleats (stretching and folding) when sheared. Although flexible, filter layer 112 and membrane 113 are not torn under constraint considered and they have a stable shape in absence of pressure and/or shear.

Finally, the chip 100 allows to inject easily a sample in a chamber able to be deformed and sheared strongly by an external mechanical device: micro-organism in the sample may be efficiently grinded, leading to mechanical lysis. Thanks to the porosity of the filter 112, separation of the internal material of micro-organisms from their outer envelope - cell membrane, capsid... - is achieved, the former flowing through the filter whereas the latter are blocked by the filter. Thus, the internal material of micro-organisms is accumulated - downstream - in the side of the lysing chamber 120 opposite the side - upstream - of lysing chamber 120 connected to the inlet port 130, where it may be collected for further analysis, such as PCR amplification.

In a second basic configuration, a second flexible filter layer 112 is introduced in the multilayer stack 110 of the chip 100 in order to maximize the volume of the injected sample. In this configuration, the second flexible filter 112 is aligned on the first flexible filter layer 112 and is in direct contact with the second double-sided adhesive, opposite with the closing surface. In this configuration, a separating layer comprising a chamber hole aligned with the flexible filter layers 112 is inserted between the two flexible filter layers 112. The separating layer may be a double-sided adhesive layer.

In the following, first and second basic configurations may be independently used in all configurations and variants. Indeed, a single flexible filter layer 112 may be replaced by a pair of flexible filter layers 112 in all configurations and variants disclosed hereafter.

In a first configuration, the closing surface is a second flexible membrane 113. In this configuration, the chip 100 is globally flexible, with a well-defined chamber delimited by the two flexible membranes 113 and, for the first basic configuration, separated in two parts by the flexible filter layer 112 and, for the second basic configuration, separated in three parts by the two flexible filter layers 112. Preferably, the second flexible membrane 113 is similar to the first flexible membrane 113 - same material and same flexibility properties.

In an embodiment of this fist configuration, the chip 100 further comprises a back cover 115 on the membrane side of the multilayer stack 110, comprising a pestle hole aligned with the lysing chamber 120. This back cover 115 provides with mechanical stability of the chip 100, allowing easier handling. The pestle hole is aligned with the lysing chamber 120, so that an external element - for instance the pestle 520 of a mechanical grinder 500 - may engage in the pestle hole so as to press and shear the flexible membranes 113 and the flexible filter layer(s) 112 of the chip 100.

In this embodiment, the chip 100 also comprises a rigid cover 114 on the second flexible membrane 113, opposite the back cover 115. The rigid cover 114 also provides with mechanical stability of the chip 100, allowing easier handling. In addition, the rigid cover 114 is a surface on which the flexible membranes 113 and the flexible filter layer 112 may be pressed by an external element, so as to increase pressure and shear. Both covers - back cover 115 and rigid cover 114 - are kept in contact with the closed multilayer stack 110 with fastening means.

Last, in this embodiment, the layers of the multilayer stack 110 and the second flexible membrane 113 comprise superimposed inlet holes, forming a small inlet chamber. In one of the double-sided adhesive layers 111 of the multilayer stack 110 of the first basic configuration, an inlet channel 116 establishes a fluidic connection between the chamber hole and the inlet hole. Considering the second basic configuration, the inlet channel 116 must be positioned in the separating layer in order to inject the sample between the two flexible filter layers 112. Last, the inlet port 130 of the chip is located on the rigid cover 114, above the inlet chamber, and thus in fluidic communication with the lysing chamber 120 through the inlet channel 116.

Finally, in this embodiment, the chip 100 is reinforced mechanically by both covers and a liquid sample deposited in the inlet port 130 may flow inside the lysing chamber 120 through the inlet chamber then the inlet channel 116. This is especially interesting to introduce the sample in the chip 100, with a micropipette for instance, where inlet port 130 and both covers help positioning the tip of micropipette correctly.

In order to collect the internal material of micro-organisms after lysis, an outlet port 140 may be on the rigid cover 114 and in fluidic communication with the lysing chamber 120.

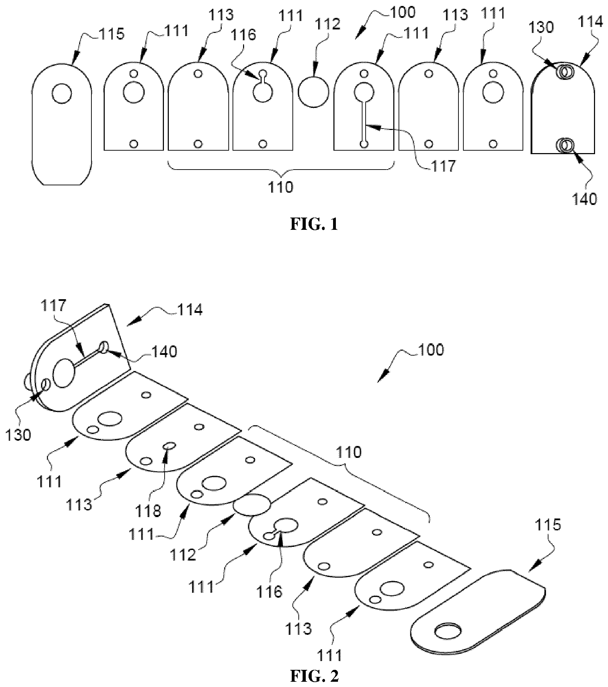

In a first variant illustrated on figure 1 in a non-limitative manner, the fluidic communication between the outlet port 140 and the lysing chamber 120 is noted A1 and designed as follows. The layers of the multilayer stack 110 and the second flexible membrane 113 comprise superimposed outlet holes, forming a small outlet chamber. In the first basic configuration, the double-sided adhesive layer 111 of the multilayer stack 110 that does not comprise the inlet channel 116, comprises an outlet channel 117 establishing a fluidic connection between the chamber hole and the outlet hole. Considering the second basic configuration, both double-sided adhesive layers 111 of the multilayer stack 110 that do not comprise the inlet channel 116 comprise an outlet channel 117 between their respective chamber holes and outlet holes. Last, the outlet port 140 of the chip is located on the rigid cover 114, above the outlet chamber, and thus in fluidic communication with the lysing chamber 120 through the outlet channel 117. In this variant, the sample may flow from the inlet port 130 to the outlet port 140 via the lysing chamber 120 and the flexible filter layer 112. Therefore, only the internal material of micro-organism may be collected at the outlet port 140.

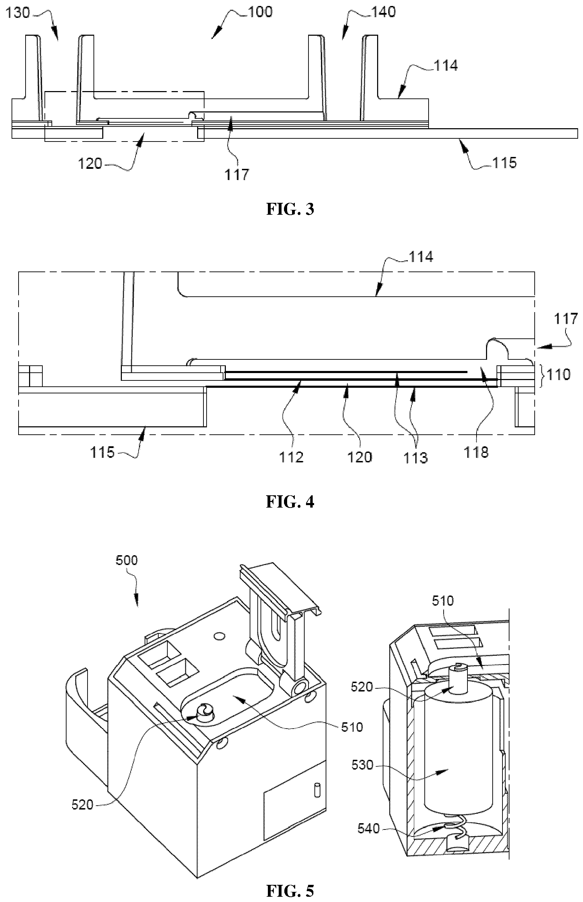

In a second variant illustrated on figure 2 in a non-limitative manner, the fluidic communication between the outlet port 140 and the lysing chamber 120 is noted B1 and designed as follows. The second flexible membrane 113 comprises a communication hole 118 located so as to be superimposed on the periphery of the lysing chamber 120. The communication hole 118 is advantageously smaller than the chamber hole, so that the second flexible membrane 113 closes almost completely the lysing chamber 120, except for a small exit through the communication hole 118. A side view of the chip 100 with the communication hole 118 is shown in figure 3 and zoomed in figure 4. In the first basic configuration, the rigid cover 114 - on the side of the second flexible membrane 113 - comprises an outlet channel 117 between the communication hole 118 and the outlet port 140. Considering the second basic configuration, the second flexible membrane 113 comprises a communication hole 118 located so as to be superimposed on the periphery of the lysing chamber 120 and the rigid cover 114 comprises an outlet channel 117 between the communication hole 118 and the outlet port 140. Moreover, in this configuration, the layers of the multilayer stack 110 and the second flexible membrane 113 comprise superimposed outlet holes, forming a small outlet chamber and the first double-sided adhesive layers 111 comprises an outlet channel 117 between the chamber hole and the outlet hole. Advantageously, the outlet channel 117 is grooved at the surface of the rigid cover 114 as represented in figures 2 and 3. Advantageously, a recess 510 may be bored in the rigid cover 114, aligned with the lysing chamber 120, so that the communication hole 118 allows fluid flow between the lysing chamber 120 and said recess 510, and so that the outlet channel 117 is grooved between said recess 510 and the outlet port 140. In this variant, internal material of micro-organism may be collected almost directly in the lysing chamber 120 This second variant has several advantages: grooving the outlet channel 117 in a rigid element is easier than designing the outlet channel 117 within an adhesive layer. And pressure drop through the outlet channel 117 in a rigid element is much lower than within an adhesive layer: thus, pressure required to introduce the sample in the lysing chamber 120 is reduced in this second variant.

In a particular aspect of this embodiment - compatible with both variants - the fastening means intended to keep both covers in contact with the closed multilayer stack 110 comprise a third double-sided adhesive layer 111 in direct contact between the back cover 115 and the multilayer stack 110, said third double-sided adhesive layer 111 comprising a pestle hole superimposed with the lysing chamber 120.

In a particular aspect of the first variant, the fastening means intended to keep both covers in contact with the closed multilayer stack 110 comprise a third double-sided adhesive layer 111 in direct contact between the back cover 115 and the multilayer stack 110 and comprising a pestle hole superimposed with the lysing chamber 120; and a fourth double-sided adhesive layer 111 in direct contact between the rigid cover 114 and the second flexible membrane 113. The fourth double-sided adhesive layer 111 further comprises an inlet hole superimposed with the inlet chamber and an outlet hole superimposed with the other outlet holes. In this specific configuration, the cohesion of the chip 100 is ensured by adhesives over the whole surface of the chip 100, leading to a very good resistance of the chip 100 during handling, sample introduction and grinding.

In a particular aspect of the second variant, the fastening means intended to keep both covers in contact with the closed multilayer stack 110 comprise a third double-sided adhesive layer 111 in direct contact between the back cover 115 and the multilayer stack 110 and comprising a pestle hole superimposed with the lysing chamber 120; and a fourth double-sided adhesive layer 111 in direct contact between the rigid cover 114 and the second flexible membrane 113. The fourth double-sided adhesive layer 111 further comprises an inlet hole superimposed with the inlet chamber and a chamber hole superimposed with the lysing chamber 120. In this specific configuration, the cohesion of the chip 100 is ensured by adhesives, leading to a very good balance between surface of adhesion - for the chip stability during handling, sample introduction and grinding - and keeping the flexible filter layer 112 free of any adhesive on the filtering area.

In the first configuration, double-sided adhesive layers 111 and flexible membranes 113 may all comprise an inlet hole and an outlet hole. Although these holes are not all required to ensure fluidic communication between inlet port 130 and lysing chamber 120, and optionally fluidic communication between lysing chamber 120 and outlet port 140, they are advantageous. Indeed, when sample is deposited in the inlet port 130 with a micropipette for instance, the tip of the micropipette may enter inside the multilayer stack 110 and become stuck by an adhesive, thus plugging the micropipette or degrading sample delivery. Inlet and outlet holes in all double-sided adhesive layers 111 and flexible membranes 113 prevent this drawback.

In a second configuration, the closing surface is a rigid cover 114. In this configuration, the chip 100 is globally rigid, with a well-defined chamber delimited by the flexible membrane 113 and the rigid cover 114, separated in two parts by the flexible filter layer 112. The rigid cover 114 provides with mechanical stability of the chip 100, allowing easier handling.

In an embodiment of the second configuration, the chip 100 further comprises a back cover 115 on side of the multilayer stack 110 opposite the rigid cover 114, comprising a pestle hole aligned with the lysing chamber 120. The pestle hole is aligned with the lysing chamber 120, so that an external element - for instance the pestle 520 of a mechanical grinder 500 - may engage in the pestle hole so as to press - against the rigid cover 114 - and shear the flexible membranes 113 and the flexible filter layer(s) 112 of the chip 100. Both covers - back cover 115 and rigid cover 114 - are kept in contact with the closed multilayer stack 110 with fastening means.

In order to collect the internal material of micro-organisms after lysis, an outlet port 140 may be on the rigid cover 114 and in fluidic communication with the lysing chamber 120.

In a first variant of this embodiment, the fluidic communication between the outlet port 140 and the lysing chamber 120 is noted A2 and designed as follows. The layers of the multilayer stack 110 comprise superimposed outlet holes, forming a small outlet chamber. In the first basic configuration, one double-sided adhesive layer 111 of the multilayer stack 110 comprises an outlet channel 117 establishing a fluidic connection between the chamber hole and the outlet hole. Considering the second basic configuration, both double-sided adhesive layers 111 of the multilayer stack 110 not positioned between the two flexible filter layers 112 comprise an outlet channel 117 between their respective chamber holes and outlet holes. Last, the outlet port 140 of the chip 100 is located on the rigid cover 114, above the outlet chamber, and thus in fluidic communication with the lysing chamber 120 through the outlet channel 117.

In a second variant of this embodiment, the fluidic communication between the outlet port 140 and the lysing chamber 120 is noted B2 and designed as follows. In the first basic configuration, the rigid cover 114 - on the side of the lysis chamber - comprises an outlet channel 117 between the chamber hole and the outlet port 140. Considering the second basic configuration, the layers of the multilayer stack 110 comprise superimposed outlet holes, forming a small outlet chamber. In this configuration, the first double-sided adhesive 111 comprises an outlet channel 117 between the chamber hole and the outlet hole and the rigid cover 114 comprises an outlet channel 117 between the chamber hole and the outlet port 14. Advantageously, the outlet channel 117 is grooved at the surface of the rigid cover 114. Advantageously, a recess 510 may be bored in the rigid cover 114, aligned with the lysing chamber 120, so that the outlet channel 117 is grooved between said recess 510 and the outlet port 140.

Last, in this embodiment - for both variants - the layers of the multilayer stack 110 comprise superimposed inlet holes, forming a small inlet chamber. In the first basic configuration, one of the double-sided adhesive layers 111 devoid of an outlet channel 117 of the multilayer stack 110 comprises an inlet channel 116 establishing a fluidic connection between the chamber hole and the inlet hole. Considering the second basic configuration, the inlet channel 116 must be comprised in the separating layer positioned between the two flexible filter layers 112. Last, the inlet port 130 of the chip 100 is located on the rigid cover 114, above the inlet chamber, and thus in fluidic communication with the lysing chamber 120 through the inlet channel 116.

In this embodiment, the sample introduced through the inlet port 130 flows into the lying chamber - upstream - and through the flexible filter layer 112. Therefore, only the internal material of micro-organism may be collected - downstream - at the outlet port 140.

In a particular aspect of this embodiment - compatible with both variants - the fastening means intended to keep both covers in contact with the closed multilayer stack 110 comprise a third double-sided adhesive layer 111 in direct contact between the back cover 115 and the multilayer stack 110, said third double-sided adhesive layer 111 comprising a pestle hole superimposed with the lysing chamber 120.

In the second configuration, double-sided adhesive layers 111 and flexible membrane 113 may all comprise an inlet hole and an outlet hole. Although these holes are not all required to ensure fluidic communication between inlet port 130 and lysing chamber 120, and optionally fluidic communication between lysing chamber 120 and outlet port 140, they are advantageous. Indeed, when sample is deposited in the inlet port 130 with a micropipette for instance, the tip of the micropipette may enter inside the multilayer stack 110 and become stuck by an adhesive, thus plugging the micropipette or degrading sample delivery. Inlet and outlet holes in all double-sided adhesive layers 111 and flexible membranes 113 prevent this drawback.

Additional layers may be introduced in the chip 100, in order to increase thickness of chambers. Other additional layers may be metallic film providing with electric conductivity and heat generation - by Joule effect - or heat conductivity. Other additional layers may be rubber films allowing to define valves on the fluidic paths. Other additional layers may be hydrophobic layers - like PTFE or surface modified plastics - to improve hydrodynamic conditions like sliding on the walls/boundaries leading to a reduction of pressure required to generate flows in the fluidic paths. Other additional layers may be spacer layers, intended to increase the volume of the lysing chamber for instance, made of any material. Such additional layers shall comprise inlet holes, outlet holes or pestle holes to maintain the fluidic connections disclosed hereabove.

In this disclosure, flexible membrane 113 may be selected in any material suitable to resist mechanical stress and shear required for micro-organism lysing. In particular flexible membrane 113 made of polyethylene terephthalate (PET) are suitable, especially PET flexible membranes having a thickness ranging from 15 µm to 150 µm.

In this disclosure, flexible filter layer 112 may be selected in any material suitable to resist mechanical stress and shear required for micro-organism lysing. In particular flexible filter layer 112 made of polycarbonate (PC) are suitable, especially PC flexible filters a porosity ranging from 0,2 µm to 1 µm. Flexible filter layer 112 have a thickness ranging from 5 µm to 50 µm.

This disclosure also relates to a system for micro-organism lysing. The system comprises a chip 100 for micro-organism lysing as disclosed hereabove. The system further comprises a grinder 500 (figure 5) comprising a platform with a recess 510 configured to receive the chip 100 for micro-organism lysing, a pestle 520 configured to be aligned with the lysing chamber 120, a motor 530 to rotate said pestle 520, and pressuring means 540 to press said pestle 520 against the lysing chamber 120. Pressuring means 540 may be spring, a hydraulic or pneumatic pressure source, hydraulic or pneumatic cylinders, motor with screw, or a heavy mass.

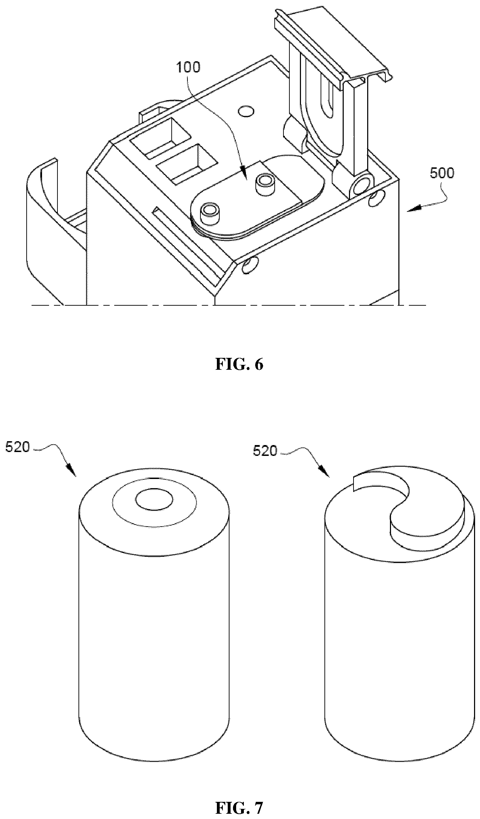

Chip 100 and grinder 500 are designed to co-operate in such a way that the chip 100 is immobilized in the grinder 500 while the pestle 520 is pressed on the lysing chamber 120 - through the pestle hole when chip 100 includes a rigid cover 114 - and rotated. A view of the chip 100 as inserted into the grinder 500 is shown in figure 6.

The grinder 500 may be designed to receive a chip 100 including covers. In this case, the pestle 520 is engaged through the pestle hole to press the lysing chamber 120. The chip 100 is immobilized in the platform by any usual means, such as a hood or slides arranged on covers.

Alternatively, the grinder 500 may be designed to receive a chip 100 without covers, i.e., a flexible chip. In this case, the geometry of the platform is configured to fit the exact shape of the flexible chip 100 and a hood is used to immobilize the chip 100. The pestle 520 then presses the lysing chamber 120 against the hood. This chip 100 without covers displaying a symmetrical configuration with respect to the flexible filter layer(s) 112, the pressure may be exerted on both side of the chip 100.

The pestle 520 may have any structure on its grinding part. It may be smooth as shown in Figure 7 (left) or irregular.

Advantageously, the grinding part of the pestle 520 is irregular, with a step shape as shown in figure 7 (right). By step shape, it is meant that the grinding part comprise a discontinuity of thickness. During lysing, pestle 520 is rotated so that pressure applied on the lysing chamber 120 is not uniform: some parts are heavily pressed, while other parts are almost not pressed. Thus, shearing is not uniform and membranes 113 are sheared, distorted, bent in an irregular manner, leading to improved lysis.

This disclosure also relates to a method for micro-organism lysing. In this method, a sample comprising micro-organisms is introduced in a chip 100 as disclosed hereabove through the inlet port 130. Then, the lysing chamber 120 of the chip 100 is pressed with a rotating pestle 520 and the internal material contained in the micro-organisms is recovered.

Advantageously, the chip 100 is placed in the recess 510 of the platform of a grinder 500, said grinder 500 comprising a pestle 520 configured to be aligned with the lysing chamber 120 of the chip 100 for micro-organism lysing, a motor 530 to rotate said pestle 520, and pressuring means 540 to press said pestle 520 against the lysing chamber 120. The pestle 520 may have any structure on its grinding part. It may be smooth or irregular. The grinder 500 disclosed hereabove is especially suitable for this method.

In an embodiment, the pressure exerted by pestle 520 is ranging from 0.02 MPa to 1.5 MPa, preferably from 0.1 MPa to 1 MPa. The pressure may be selected according to the exact chip geometry, the type of flexible membrane 113 and filters 112, and also to the type of micro-organisms to be lysed. The pressure exerted by pestle 520 is the force applied by pestle 520 divided by the surface of pestle grinding part. The force applied is ranging from 1 N to 24 N, for a surface of pestle grinding part ranging from 15 mm² to 50 mm².

In an embodiment, the rotational speed of pestle 520 is ranging from 30 rpm to 200 rpm, preferably from 50 rpm to 150 rpm. Rotational speed of pestle 520 of 60 rpm, 90 rpm and 120 rpm are especially adequate. The rotational speed may be selected according to the exact chip geometry, the type of flexible membrane 113 and filters 112, and also to the type of micro-organisms to be lysed.

In an embodiment, the duration of grinding is ranging from 30 s to 10 min. A short grinding duration is preferable, to improve output of system for micro-organism lysing. Duration of grinding or 1 min, 2 min, 3 min are especially adequate.

Appropriate conditions for lysing Gram-negative *salmonella enterica* are a grinding time of 1 min at 60 rpm with a pestle force of 12 N, with chip design V as disclosed below.

Appropriate conditions for lysing Gram-positive *listeria monocytogenes* are a grinding time of 2 min at 60 rpm with a pestle force of 12 N, with chip design V as disclosed below.

Appropriate conditions for lysing *saccharomyces cerevisiae* yeasts are a grinding time of 1 min at 120 rpm with a pestle force of 20 N, with chip design V as disclosed below.

### BRIEF DESCRIPTION OF THE DRAWINGS

**Figure 1** is an exploded view of the structure of a chip according to a first variant of the first configuration of the disclosure.
**Figure 2** is an exploded view of the structure of a chip according to a second variant of the first configuration of the disclosure.
**Figure 3** is a sectional side view of a chip according to a second variant of the first configuration of the disclosure.
**Figure 4** is an enlargement of Figure 3.
**Figure 5** is a view of a grinder: external view on left, sectional view on the right to visualize motor and pressuring means.
**Figure 6** shows the association of a grinder and a chip forming a system for micro-organism lysing.
**Figure 7** is a view of pestles with a smooth grinding part (left) and with an irregular - step shaped - grinding part (right).

### EXAMPLES

The present invention is further illustrated by the following examples.

Various chips are used, with the following specifications:

Chip design I according to first configuration: elements are laid in this order - direct contact - from top to bottom, as shown in figure 1: rigid cover 114 with inlet port 130 and outlet port 140; double-sided adhesive 111 (fastening means); flexible membrane 113; double-sided adhesive 111 with outlet channel 117; flexible filter 112; double-sided adhesive 111 with inlet channel 116; flexible membrane 113; double-sided adhesive 111 (fastening means) and back cover 115. All double-sided adhesive layers 111 and flexible membrane 113 have inlet hole and outlet hole. In design I; the volume of the lysing chamber 120 upstream of the filter 112 is about 30 µL.

Chip design II according to first configuration: elements are laid in this order - direct contact - from top to bottom: rigid cover 114 with inlet port 130 and outlet port 140 (closing surface); double-sided adhesive 111 (fastening means); flexible membrane 113; double-sided adhesive 111; spacer layer; double-sided adhesive 111 with outlet channel 117; flexible filter 112; double-sided adhesive 111 with inlet channel 116; flexible membrane 113; double-sided adhesive 111 (fastening means) and back cover 115. All double-sided adhesive layers 111; spacer layer and flexible membrane 113 have inlet hole and outlet hole. In design II; the spacer layer and additional double-sided adhesive layer define a thicker lysing chamber: volume of lysing chamber 120 upstream of the filter 112 about 30 µL.

Chip design III according to second configuration: elements are laid in this order - direct contact - from top to bottom: rigid cover 114 with inlet port 130 and outlet port 140 (closing surface); double-sided adhesive 111 (fastening means); spacer layer; double-sided adhesive 111 with outlet channel 117; flexible filter 112; double-sided adhesive 111 with inlet channel 116; flexible membrane 113; double-sided adhesive 111 (fastening means) and back cover 115. All double-sided adhesive layers 111; spacer layer and flexible membrane 113 have inlet hole and outlet hole. In design III; the volume of lysing chamber upstream of the filter 112 is similar to design II; but the lysing chamber 120 is closed by the rigid cover 114 instead of a flexible membrane 113.

Chip design IV according to second configuration: elements are laid in this order - direct contact - from top to bottom: rigid cover 114 with inlet port 130, outlet port 140 (closing surface) and outlet channel 117 grooved; double-sided adhesive 111; flexible filter 112; double-sided adhesive 111 with inlet channel 116; flexible membrane 113; double-sided adhesive 111 (fastening means) and back cover 115. All double-sided adhesive layers 111 and flexible membrane 113 have inlet hole and outlet hole. In design IV; the volume of lysing chamber 120 upstream of the filter 112 is similar to design I; but the lysing chamber 120 is closed by the rigid cover 114 instead of a flexible membrane 113.

Chip design V according to first configuration: elements are laid in this order - direct contact - from top to bottom: rigid cover 114 with inlet port 130, outlet port 140 and an outlet channel 117 grooved; double-sided adhesive 111 (fastening means); flexible membrane 113 with communication hole 118; double-sided adhesive 111; flexible filter 112; double-sided adhesive 111 with inlet channel 116; flexible membrane 113; double-sided adhesive 111 (fastening means) and back cover 115 (figure 2). All double-sided adhesive layers 111 and flexible membrane 113 have inlet hole and outlet hole. In design V; the volume of lysing chamber 120 upstream of the filter 112 is similar to design I; but the lysing chamber 120 is fluidically connected to the outlet port 140 through a hole in a flexible membrane 113 and the outlet channel 117 grooved on the rigid cover 114. Figure 3 is a sectional side view of chip design V, with an enlargement in figure 4 to see flexible membranes 113 and flexible filter 112.

All chips have a chamber hole of diameter 10 mm, inlet holes and outlet holes of diameter 4 mm. Double-sided adhesive thickness is 150 µm. Flexible membrane 113 thickness is 50 µm. Flexible filter 112 is a disc of diameter 13 mm and thickness 10 µm. Rigid cover 114 and back cover 115 are in polycarbonate, with a thickness of 2 mm, resp. 1 mm.

Grinding protocol used in these examples have the following specifications. Chip is inserted in the grinder 500 as shown on figure 6. Then pestle 520 - step shaped surface of grinding part about 25 mm² - is brought in rotation at rotational speed of 60 rpm and pressed against the lysing chamber 120 with a force of 12 N for 2 min - pressure applied by the pestle 520 is about 0.5 MPa.

Two types of protocols have been used.

### PCR protocol

A micro-organism is cultured overnight in suitable conditions of temperature, humidity and medium. Then, micro-organisms are transferred into Ringer's solution and diluted to reach a colony forming unit (CFU) of 10⁴ CFU/mL. Then 25 mL of said solution is added to 225 mL of BPW culture medium - buffered peptone water - and 25 g of a mixture of frozen vegetables into a culture bag, yielding a 10³ CFU/mL sample. The sample is homogenized using a Stomacher - BagMixer^{®} 400 from Interscience, 8 coups/s, 400W power - during 1 min. This process aims at preparing a sample representative of a food product containing 10³ CFU/mL of a known micro-organism.

1 mL of said sample is introduced in a chip. Then, 1 mL of rinsing buffer is introduced in the chip. The chip is placed in the grinder 500 for 1 min, with determined pestle 520, pressure and rotational speed. A 20 µL sample is collected from the lysing chamber 120 and tested by Polymerase Chain Reaction (PCR) in conditions suitable for the nucleic acid of interest, allowing for detection of the micro-organisms. Cycle threshold (Ct) is then reported. The lowest the Ct value, the best the lysis performance.

### DNA dosing protocol

A micro-organism is cultured overnight in suitable conditions of temperature, humidity and medium. Then, micro-organisms are transferred into Ringer's solution and diluted to reach a colony forming unit (CFU) of 10⁷ CFU/mL. 1 mL of said sample is introduced in a chip. Then, 1 mL of rinsing buffer is introduced in the chip. The chip is placed in the grinder 500 for 1 min, with determined pestle 520, pressure and rotational speed. A 20 µL sample is collected from the lysing chamber 120. DNA content is then measured according to state-of-the-art methods (in ng/µL), as well as Cycle threshold (Ct). Note that sample is much more concentrated in DNA protocol (10⁷ CFU/mL) as compared to PCR protocol (10³ CFU/mL): Ct in DNA protocol is thus much lower than Ct in PCR protocol.

The following tables show experimental results. For each experiment, measurements were reproduced three or five times, result is an average. When results are highly dispersed, an indication of dispersion is given.

Various designs and parameters have been used in PCR protocol (10³ CFU/mL), showing a overall a very good performance for lysing and detection of micro-organisms. It appears that polycarbonate-based filters (F2 and F5) give better results, as well as PET membranes (M2 and M3). Chip designs I and V also show the best results, with Ct 31 and 32, when filter F5 and membrane M3 are used.

**Table 1**

| | Chip Design | Flex. Membrane | Flex Filter | Grinding (rpm/N/min) | Micro-organism | Ct | Protocol |
|---|---|---|---|---|---|---|---|
| 1 | V | M3 | F1 | 60/12/2 | µO1 | 36 | PCR |
| 2 | V | M3 | F2 | 60/12/2 | µO1 | 33 | PCR |
| 3 | V | M3 | F3 | 60/12/2 | µO1 | >39 | PCR |
| 4 | V | M3 | F4 | 60/12/2 | µO1 | >39 | PCR |
| 5 | V | M3 | F5 | 60/12/2 | µO1 | 32 | PCR |
| 11 | V | M1 | F5 | 60/12/2 | µO1 | 35.7±2 | PCR |
| 12 | V | M2 | F5 | 60/12/2 | µO1 | 34.1±0.5 | PCR |
| 13 | V | M3 | F5 | 60/12/2 | µO1 | 34.2±0.5 | PCR |
| 21 | I | M3 | F5 | 60/12/2 | µO1 | 31 | PCR |
| 22 | II | M3 | F5 | 60/12/2 | µO1 | 33 | PCR |
| 23 | III | M3 | F5 | 60/12/2 | µO1 | 39 (±4) | PCR |
| 24 | IV | M3 | F5 | 60/12/2 | µO1 | 33 | PCR |

Comparative experiments have been run, using the DNA protocol (10⁷ CFU/mL). Chip V in association with filter F5 and membrane M3 shows a much better sensibility as compared to prior art methods. DNA retrieved after lysis is at least 5 times more concentrated.

**Table 2**

| | Chip Design | Flex. Membrane | Flex Filter | Grinding (rpm/N/min) | Micro-organism | [DNA] Ct | Protocol |
|---|---|---|---|---|---|---|---|
| 31 | V | M3 | F5 | 60/12/2 | µO1 | 1,1±0.3 14.5 | DNA |
| 32 | WO 2015181743 method | | | | µO1 | 0.25±0.05 18 | DNA |
| 33 | Qiagen DNeasy Powerlyzer kit | | | | µO1 | 0.12±0.05 18 | DNA |
| 34 | Thermofisher Suretect kit | | | | µO1 | 0.02 23 | DNA |

### Filters:

F1 - Sartorius cellulose acetate 0,45µm (SCA) ref:11106 13 N
F2 - Polycarbonate Isopore Membrane Filters 0,4µm (IMF 4) ref: HTBP01300
F3 - Mixed cellulose Ester Membrane 0,65µm (CEM) ref: DAWP01300
F4 - Mixed cellulose Ester Membrane Filters 0,45µm (MF) ref: HAWP02500
F5 - Polycarbonate Isopore Membrane Filters 0,6µm (IMF 6) ref: DTTP01300

### Membranes:

M1 - Microamp film (PMMA 35 µm thick PMMA with one adhesive face)
M2 - PET film 23 µm thick.
M3 - PET film 50 µm thick.

For micro-organism *Listeria monocytogenes* ATCC 13932, noted µO1, PCR testing was done with a Suretect kit provided by Thermofisher, ref. PT0300A. DNA dosing was done with Invitrogen^{™} Qubit^{™} dsDNA HS Assay Kit, after fluorescence measurements on Invitrogen^{™} Fluoromètre Qubit^{™} 4 reader.

### Influence of pestle type

With chip design I in the DNA protocol, the type of pestle 520 is compared. With smooth pestle 520 of figure 7 (left), total DNA concentration is measured at 0.78 (±0.02) ng/µL. With step shaped pestle 520 of figure 7 (right), total DNA concentration is measured at 5.2 (±0.4) ng/µL. The irregular shape of grinding part of pestle 520 increases noticeably the amount of DNA released, evidencing a more quantitative lysis of micro-organisms.

### NUMERICAL REFERENCES

100 - Chip / 110 - Multilayer stack / 111 - Double sided adhesive layer / 112 - Flexible filter layer / 113 - Flexible membrane / 114 - Rigid cover / 115 - Back cover / 116 - Inlet channel / 117 - Outlet channel / 118 - Communication hole / 120 - Lysing chamber / 130 - Inlet port / 140 - Outlet Port / 500 - Grinder / 510 - Recess / 520 - Pestle / 530 - Motor / 540 - Pressuring means

## Claims

1. A chip (100) for micro-organism lysing comprising
a) a multilayer stack (110) comprising
i. a first double-sided adhesive layer (111) comprising a chamber hole;
ii. a flexible filter layer (112) in direct contact with the first double-sided adhesive layer (111) and covering the chamber hole;
iii. a second double-sided adhesive layer (111) in direct contact with the flexible filter layer (112), said second double-sided adhesive layer (111) comprising a chamber hole; and
iv. a flexible membrane (113) on the first double-sided adhesive layer (111), opposite the flexible filter (112);
wherein all chamber holes are superimposed to form a lysing chamber (120); and
b) a closing surface on the second double-sided adhesive layer (111) of the multilayer stack (110), opposite the flexible membrane (113);
c) an inlet port (130) in fluidic communication with the lysing chamber (120).

2. The chip (100) for micro-organism lysing according to claim **1** wherein the closing surface is a second flexible membrane (113).

3. The chip (100) for micro-organism lysing according to claim **2** further comprising
a) a back cover (115) on the flexible membrane side of the multilayer stack (110), comprising a pestle hole aligned with the lysing chamber (120);
b) a rigid cover (114) on the second flexible membrane (113), opposite the back cover (120); and
c) fastening means to maintain rigid cover (114) and back cover (115) in contact with the closed multilayer stack (110);
wherein the inlet port (130) is on the rigid cover (114); the fluidic communication being arranged by superimposed inlet holes in the layers of the multilayer stack (110) and in the second flexible membrane (113) forming an inlet chamber and an inlet channel (116) between the chamber hole and the inlet hole of one of the double-sided adhesive layers (111).

4. The chip (100) for micro-organism lysing according to claim **3** further comprising an outlet port (140) in fluidic communication with the lysing chamber (120), the outlet port (140) being on the rigid cover (114); and
i. the fluidic communication, noted A1, being arranged by superimposed outlet holes in the layers of the multilayer stack (110) and in the second flexible membrane (113) forming an outlet chamber; and an outlet channel (117) between the chamber hole and the outlet hole of the double-sided adhesive layer (111) devoid of an inlet channel (116); or
ii. the fluidic communication, noted B1, being arranged by a communication hole (118) in the second flexible membrane (113), said communication hole (118) being superimposed on the periphery of the lysing chamber (120); and an outlet channel (117) between the communication hole and the outlet port (140) in the rigid cover (114).

5. The chip (100) for micro-organism lysing according to claim **3** or **4,** wherein fastening means comprise a third double-sided adhesive layer (111) in direct contact between the back cover (115) and the multilayer stack (110), said third double-sided adhesive layer (111) comprising a pestle hole superimposed with the lysing chamber (120).

6. The chip (100) for micro-organism lysing according to claim **4,** wherein fastening means comprise a third double-sided adhesive layer (111) in direct contact between the back cover (115) and the multilayer stack (110) and comprising a pestle hole superimposed with the lysing chamber (120); and a fourth double-sided adhesive layer (111) in direct contact between the rigid cover (114) and the second flexible membrane (113) and comprising an inlet hole superimposed with the inlet chamber and
i. an outlet hole superimposed with the other outlet holes for fluidic communication A1; or
ii. a chamber hole superimposed with the lysing chamber for fluidic communication B1.

7. The chip (100) for micro-organism lysing according to claim **1** wherein the closing surface is a rigid cover (114).

8. The chip (100) for micro-organism lysing according to claim **7** further comprising
a) a back cover (115) on side of the multilayer stack (110) opposite the rigid cover (114), comprising a pestle hole aligned with the lysing chamber (120);
b) fastening means to maintain back cover (115) in contact with the multilayer stack (110);
c) an outlet port (140) in fluidic communication with the lysing chamber (120), the outlet port (140) being on the rigid cover (114); and
i. the fluidic communication, noted A2, being arranged by superimposed outlet holes in the layers of the multilayer stack (110) forming an outlet chamber; and an outlet channel (117) between the chamber hole and the outlet hole of one of the double-sided adhesive layers (111); or
ii. the fluidic communication, noted B2, being arranged by an outlet channel (117) between the chamber hole and the outlet port (140) in the rigid cover (114); and wherein the inlet port (130) is on the rigid cover (114); the fluidic communication being arranged by superimposed inlet holes in the layers of the multilayer stack (110) forming an inlet chamber and an inlet channel (116) between the chamber hole and the inlet hole of a double-sided adhesive layer (111) devoid of an outlet channel (117).

9. The chip (100) for micro-organism lysing according to any one of claims **1** to **8,** wherein flexible membranes (113) are polyethylene terephthalate membranes, preferably flexible membranes (113) have a thickness ranging from 15 µm to 150 µm.

10. The chip (100) for micro-organism lysing according to any one of claims **1** to **9,** wherein flexible filter layer (112) is a polycarbonate layer, preferably flexible filter (112) layer has a porosity ranging from 0,2 µm to 1 µm.

11. A system for micro-organism lysing comprising
a. a chip (100) for micro-organism lysing according to any one of claims **1** to **10,**
b. a grinder (500) comprising
i. a platform with a recess (510) configured to receive the chip (100) for micro-organism lysing;
ii. a pestle (520) configured to be aligned with the lysing chamber (120);
iii. a motor (530) to rotate said pestle (520); and
iv. pressuring means (540) to press said pestle (520) against the lysing chamber (120).

12. The system for micro-organism lysing according to claim **11,** wherein the pestle grinding part comprises a step.

13. A method for micro-organism lysing comprising
a. Introducing a sample comprising micro-organisms via the inlet import (130) in a chip (100) for micro-organism lysing according to any one of claims **1** to **10;**
b. Pressing the lysing chamber (120) with a rotating pestle (520); and
c. Recovering the content of cells.

14. The method for micro-organism lysing according to claim **13,** wherein the chip (100) for micro-organism lysing is placed in the recess of the platform (510) of a grinder (500), said grinder (500) comprising
i. a pestle (520) configured to be aligned with the lysing chamber (120) of the chip (100) for micro-organism lysing;
ii. a motor (530) to rotate said pestle (520); and
iii. pressuring means (540) to press said pestle (520) against the lysing chamber (120).

15. The method for micro-organism lysing according to claim **13,** wherein the force of pestle (520) is ranging from 1 N to 24 N and/or the rotational speed of pestle (520) is ranging from 30 rpm to 150 rpm.
